(19) Europäisches Patentamt
European Patent Office
Office européen des brevets

(11) Veröffentlichungsnummer: **0 040 350**
**B1**

# (12) EUROPÄISCHE PATENTSCHRIFT

(45) Veröffentlichungstag der Patentschrift: **21.09.83**

(51) Int. Cl.[3]: **C 07 D 249/08,** C 07 D 233/60, A 01 N 43/50, A 01 N 43/64

(21) Anmeldenummer: **81103348.9**

(22) Anmeldetag: **04.05.81**

(54) **Neue Azolverbindungen, ihre Herstellung, ihre Verwendung zur Pflanzenbehandlung und Mittel dafür.**

(30) Priorität: **19.05.80 DE 3019049**

(43) Veröffentlichungstag der Anmeldung: **25.11.81 Patentblatt 81/47**

(45) Bekanntmachung des Hinweises auf die Patenterteilung: **21.09.83 Patentblatt 83/38**

(84) Benannte Vertragsstaaten: **AT BE CH DE FR GB IT LI NL SE**

(56) Entgegenhaltungen:
**AT-B-334 686**
**AT-B-340 410**
**DE-A-2 431 073**
**DE-A-2 800 544**
**DE-A-2 838 847**
**DE-A-2 842 137**

(73) Patentinhaber: **BASF Aktiengesellschaft, Carl-Bosch-Strasse 38, D-6700 Ludwigshafen (DE)**

(72) Erfinder: **Sauter, Hubert, Dr., Neckarpromenade, D-6800 Mannheim (DE)**
Erfinder: **Ammermann, Eberhard, Dr., Sachsenstrasse 3, D-6700 Ludwigshafen (DE)**
Erfinder: **Rentzea, Costin, Dr., Neuenheimer Landstrasse 72, D-6900 Heidelberg (DE)**
Erfinder: **Zeeh, Bernd, Dr., Thorwaldsenstrasse 5, D-6700 Ludwigshafen (DE)**
Erfinder: **Jung, Johann, Dr., Hardenburgstrasse 19, D-6703 Limburgerhof (DE)**
Erfinder: **Pommer, Ernst-Heinrich, Dr., Berliner Platz 7, D-6703 Limburgerhof (DE)**

**EP 0 040 350 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue Azolverbindungen, ihre Herstellung, ihre Verwendung zur Pflanzenbehandlung und Mittel dafür

Die vorliegende Erfindung betrifft neue, wertvolle Azolverbindungen, Verfahren zu ihrer Herstellung, diese Verbindungen enthaltende Pflanzenbehandlungsmittel und deren Verwendung.

Es ist bekannt, dass Imidazolderivate, zum Beispiel das 1-(2,4-Dichlorphenyl-β-allylethylether)-imidazol (DE-OS 2 063 857), und Triazolderivate, zum Beispiel das 2,2-Dimethyl-4-(1,2,4-triazol-1-yl)-5-phenyl-pentanon-(3) (DE-OS 2 638 470), eine gute fungizide Wirksamkeit zeigen. Die Wirkung ist jedoch bei niedrigen Aufwandmengen und Anwendungskonzentrationen nicht immer befriedigend. Darüber hinaus ist die fungitoxische Wirkung oft mit einer hohen Phytotoxizität verbunden, so dass in den für die Bekämpfung von Pilzen im Pflanzenschutz, beispielsweise bei der Bekämpfung von Rostpilzen notwendigen Konzentrationen auch die Kulturpflanzen geschädigt werden. Aus diesen Gründen sind sie für den Gebrauch als Pflanzenschutzmittel zur Bekämpfung von Pilzen nicht immer und nicht bei allen Pflanzenarten geeignet. Weiter sind fungizid wirksame Triazolyl-phenoxy-methan-Derivate bekannt (DE-OS 24 31 073, DE-OS 28 42 137 und AT-PS 334 686).

Es ist ferner bekannt, dass quartäre Ammoniumverbindungen wie Chlorcholinchlorid (CCC) (J. Biol. Chem. 235, 475 (1960)), und gewisse Triazolderivate, zum Beispiel das 3-(1,2,4-Triazol-1-yl)-1-(4-chlorphenyl)-4,4-dimethylpentanon-(1) (DE-OS 2 739 352), einen Einfluss auf das Wachstum von Kulturpflanzen haben und als Pflanzenwachstumsregulatoren verwendet werden können. Die pflanzenwachstumsregulierende Wirkung dieser Verbindungen ist jedoch, besonders bei niedrigen Aufwandmengen, nicht immer befriedigend. Es wurden nun neue Verbindungen gefunden, die eine sehr hohe fungitoxische Wirkung bei ausgezeichneter Pflanzenverträglichkeit aufweisen. Die neuen Verbindungen zeigen darüber hinaus auch bei niedrigen Aufwandmengen starke pflanzenwachstumsregulierende Wirkungen bei sehr guter Pflanzenverträglichkeit.

Gegenstand der Erfindung sind neue Azolverbindungen der Formel I

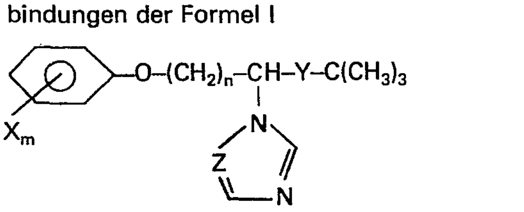

(I),

in der

X für Wasserstoff, Halogen, $C_1$–$C_4$-Alkyl, $C_1$–$C_4$-Alkoxy, Trifluormethyl oder Phenyl steht und m eine ganze Zahl von 1 bis 5 ist, wobei die einzelnen Gruppen X gleich oder verschieden sind, wenn m grösser als 1 ist,

n eine ganze Zahl von 2 bis 5 ist,

Z für N oder CH steht und

Y für CO oder die Gruppe $CR^1OR^2$ steht, in welcher $R^1$ Wassterstoff oder $C_1$–$C_4$-Alkyl bedeutet und $R^2$ Wasserstoff, $C_1$–$C_4$-Alkyl, $C_3$–$C_4$-Alkenyl, $C_2$–$C_4$-Alkinyl oder $C_1$–$C_4$-Alkanoyl bedeutet, sowie deren Säureadditionssalze und Metallkomplexsalze.

In den erfindungsgemässen Verbindungen der Formel I ist das azolylsubstituierte Kohlenstoffatom chiral; die Wirkstoffe fallen demgemäss als Enantiomerengemisch an, die in die optisch aktiven Verbindungen getrennt werden können. Im Falle der Alkohole, Ether oder Ester ($Y = CR^1OR^2$) treten durch das zusätzliche chirale Zentrum Diastereomerengemische auf, die in üblicher Weise, z.B. durch Kristallisation oder Chromatographie in die einzelnen Komponenten, getrennt werden können. Für die Anwendung der erfindungsgemässen Verbindungen als Fungizide oder Pflanzenwachstumsregulatoren ist jedoch eine Trennung der Enantiomeren oder Diastereomeren normalerweise nicht erforderlich.

Der Phenoxyrest kann beispielsweise folgendermassen durch $X_m$ substituiert sein:

| | |
|---|---|
| Wasserstoff | 3-Methyl-, |
| 2-Fluor-, | 4-Methyl-, |
| 4-Fluor-, | 3-tert.-Butyl-, |
| 2-Chlor-, | 4-tert.-Butyl-, |
| 3-Chlor-, | 2-Methoxy-, |
| 4-Chlor-, | 3-Methoxy-, |
| 4-Brom-, | 4-Methoxy-, |
| 2,4-Dichlor-, | 3,5-Dimethoxy-, |
| 2,4,6-Trichlor-, | 3-n-Butoxy-, |
| 3,5-Dichlor-, | 4-n-Butoxy-, |
| 2-Chlor-4-phenyl-, | 2-Methoxy-4-methyl-, |
| 2-Methyl-4-chlor-, | 3-Trifluormethyl |
| 2-Methyl-, | |

$R^1$ bedeutet beispielsweise Wasserstoff, Methyl, Ethyl, n-Propyl, Isopropyl, n-Butyl.

$R^2$ bedeutet beispielsweise Wasserstoff, Methyl, Ethyl, n-Propyl, Prop-2-enyl-(1), Prop-2-inyl-(1), n-Butyl, 2-Methylprop-2-enyl-(1), Acetyl, Propionyl, Butyryl, Isobutyryl.

Geeignete Säureadditionssalze sind beispielsweise die Bromide, Sulfate, Nitrate, Phosphate, Oxalate oder Dodecylbenzolsulfonate. Die Wirksamkeit der Salze geht auf das Kation zurück, so dass die Wahl des Anions beliebig ist.

Geeignete Metallkomplexe sind Verbindungen der Formel

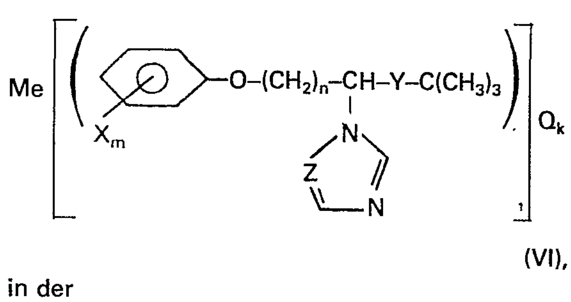

(VI),

in der

$X_m$, n, Z und Y die oben angegebene Bedeutung haben und Me ein Metall, z.B. Kupfer, Zink, Zinn, Mangan, Eisen, Cobalt oder Nickel bedeutet.

Q für das Anion einer anorganischen Säure steht, z.B. Salzsäure, Schwefelsäure, Phosphorsäure oder Bromwasserstoffsäure und 1 und k 1, 2, 3 oder 4 bedeuten.

Gegenstand der Erfindung ist weiter ein Verfahren zur Herstellung der Azolverbindungen der Formel I gemäss Anspruch 1, dadurch gekennzeichnet, dass man Ketone der Formel II

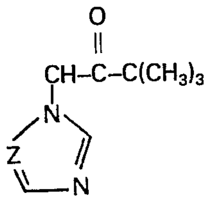

(II),

in der Z die angegebenen Bedeutungen hat, oder deren Alkalienolate mit einem ω-Aryloxyalkylhalogenid der Formel III

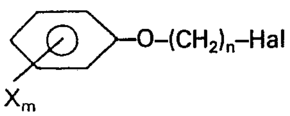

(III),

in der X, m und n die unter Anspruch 1 angegebenen Bedeutungen haben und Hal für Chlor, Brom oder Iod steht, gegebenenfalls in Gegenwart eines Lösungs- oder Verdünnungsmittels und/oder anorganischer oder organischer Basen bei Temperaturen zwischen 0 und 100 °C, umsetzt und die so erhaltenen Verbindungen der Formel I, in denen Y eine CO-Gruppe darstellt, gegebenenfalls anschliessend durch Einwirkung eines komplexen Hydrids oder durch Einwirkung von Wasserstoff in Gegenwart eines Hydrierungskatalysators und gegebenenfalls in Gegenwart eines Lösungs- oder Verdünnungsmittels bei Temperaturen zwischen 0 und 100 °C zu sekundären Alkoholen der Formel I, in denen Y eine CHOH-Gruppe darstellt, reduziert oder mit einer Grignardverbindung der Formel IV

$R^1$–MgHal IV,

in der $R^1$ für $C_1$–$C_4$–Alkyl steht und in der Hal Chlor, Brom oder Iod bedeutet, gegebenenfalls in Gegenwart eines Lösungs- oder Verdünnungsmittels und gegebenenfalls in Gegenwart eines Magnesium- oder Tetraalkylammoniumhalogenids bei Temperaturen zwischen 0 und 100 °C umsetzt und die so entstandenen Alkoholate anschliessend zu den tertiären Alkoholen hydrolysiert und die so erhaltenen sekundären oder tertiären Alkoholate – falls gewünscht – mit einem $C_1$–$C_4$-Alkanoylchlorid oder einem $C_1$–$C_4$-Alkanoylhydrid gegebenenfalls in Gegenwart eines Lösungs- oder Verdünnungsmittels und/oder einer anorganischen oder organischen Base und/oder eines Acylierungskatalysators bei Temperaturen zwischen 0 und 100 °C oder diese oder deren Alkali- oder quartären Ammoniumsalze mit einem Alkylierungsmittel der Formel V

L–$R^2$ V,

in der $R^2$ $C_1$–$C_4$–Alkyl, $C_2$–$C_4$–Alkenyl oder $C_2$–$C_4$–Alkinyl bedeutet und L für eine nucleophile verdrängbare Abgangsgruppe steht, gegebenenfalls in Gegenwart eines Lösungs- oder Verdünnungsmittels und/oder anorganischer oder organischer Basen und/oder eines Reaktionsbeschleunigers bei Temperaturen zwischen 0 und 100 °C umsetzt und die so erhaltenen Verbindungen der Formel I gegebenenfalls anschliessend in ihre für Pflanzen verträglichen Säureadditionssalze oder Metallkomplexe überführt.

Das Verfahren zur Herstellung der Ketone der Formel I, in denen R eine CO-Gruppe darstellt, besteht darin, dass man bekannte Ketone der Formel II (DE-OS 2 638 470) oder deren Alkalienolate mit ω-Aryloxyalkylhalogeniden III, gegebenenfalls in Gegenwart einer Base und/oder eines Lösungs- oder Verdünnungsmittels, zu den erfindungsgemässen Ketonen der Formel Ia alkyliert.

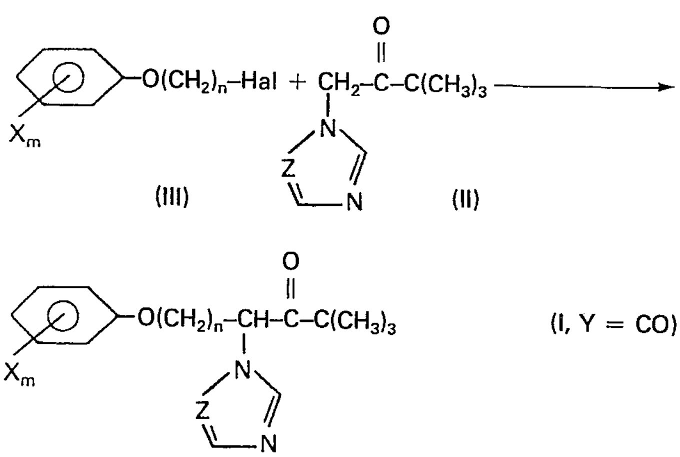

(III) (II)

(I, Y = CO)

Hierzu können die Ketone II zunächst zu den Alkalienolaten metalliert werden, indem man sie vorzugsweise in Gegenwart eines polaren aprotischen Lösungsmittels wie Dimethylformamid, Acetonitril oder Tetrahydrofuran mit 0,8 bis 1,2 Äquivalenten, bevorzugt 1,0 Äquivalenten, eines Metallierungsreagens wie Natriumhydrid, Lithiumdiisopropylamid oder n-Butyllithium bei 0

bis 100 °C, vorzugsweise bei 10° bis 50 °C, umsetzt. Nach anschliessender Zugabe von 0,8 bis 2,0, bevorzugt 1,0 Äquivalenten des jeweiligen ω-Aryloxyalkylhalogenids der Formel III erhält man bei Reaktionstemperaturen zwischen 0° und 100 °C, bevorzugt bei 5° bis 30 °C, die Ketone der Formel I.

Eine Variante dieses Verfahrens besteht darin, dass man die Ketone II in Gegenwart von 0,8 bis 1,2, bevorzugt 1,0 Äquivalenten einer Base wie z.B. Kalium-tert.-butoxid, Natriummethoxid oder Kaliumhydroxid, mit den ω-Aryloxyalkylhalogeniden III umsetzt, wobei man zweckmässigerweise in Gegenwart eines Lösungs- oder Verdünnungsmittels bei Temperaturen zwischen 0 °C und 100 °C, bevorzugt bei 5° bis 50 °C, arbeitet.

Als Lösungsmittel oder Verdünnungsmittel kommen hier wiederum dipolare aprotische Lösungsmittel in Frage, aber auch Alkohole wie Methanol oder tert.-Butanol.

Die ω-Aryloxyhalogenide III sind bekannte Verbindungen oder können leicht nach bekannten Verfahren hergestellt werden, z.B. durch Monoalkylierung von Phenolen mit aliphatischen Dihalogenalkanen, z.B. mit 1,2-Dibromethan, 1,3-Dichlorpropan, 1,4-Dibrombutan oder 1,5-Dibrompentan (siehe Houben-Weyl, Methoden der Organischen Chemie, Band 6/3, S. 54–59, Thieme-Verlag, Stuttgart 1965, sowie Beispiel 1b und 5).

Das Verfahren zur Herstellung der sekundären Alkohole der Formel I, in der Y die Gruppe

OH
I
–C– darstellt, besteht darin, dass man die Ketone
I
H

der Formel I, in denen eine CO-Gruppe ist, einer Reduktion unterwirft, z.B. durch Einwirkung von komplexen Hydriden, bevorzugt Natriumborhydrid in Gegenwart eines polaren Lösungsmittels, z.B. eines Alkohols, bevorzugt Methanol oder Ethanol, bei Temperaturen zwischen 0 bis 100 °C und anschliessender Hydrolyse mit wässrigen Basen oder Säuren oder durch Einwirkung von Wasserstoff in Gegenwart eines Hydrierungskatalysators wie Platin oder Raney-Nickel und in Gegenwart eines polaren Lösungsmittels wie Methanol, Ethanol oder Ethylacetat bei Temperaturen zwischen 20 und 100 °C und Drücken von 1 bis 100 bar:

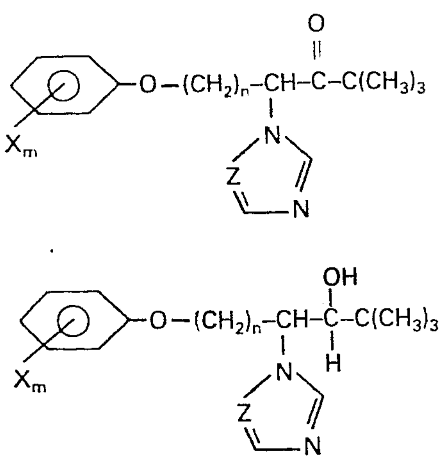

(I, Y = CO)

(I, Y = –C(OH)(H)–)

Das Verfahren zur Herstellung der tertiären Alkohole der

OH
I
Formel I (Y=– C–, worin $R^1$ für $C_1$–$C_4$-Alkyl, nicht
I
$R^1$

aber für Wasserstoff steht) besteht darin, dass man die Ketone der Formel I (Y=CO) mit 0,8 bis 1,2 Äquivalenten einer Grignardverbindung der Formel IV

$R^1$–MgHal IV,

in der $R^1$ $C_1$–$C_4$–Alkyl und Hal Chlor, Brom oder Iod bedeutet, vorzugsweise in Abwesenheit eines Lösungsmittels und gegebenenfalls in Anwesenheit eines ausbeutesteigernden Salzes, umsetzt:

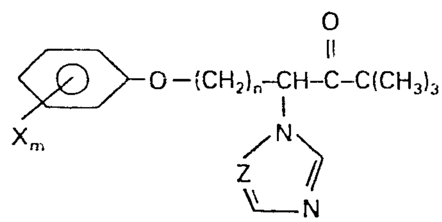

(I, Y = CO)

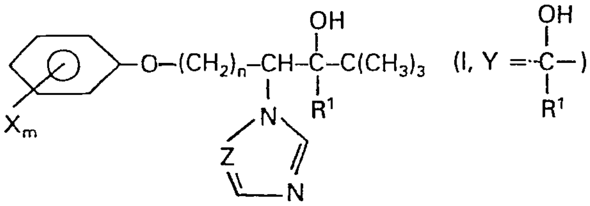

Als Lösungsmittel kommen bevorzugt Ether in Frage, wie Diethylether, Di-n-propylether, Tetrahydrofuran oder Anisol, ferner tertiäre Amine wie N,N-Diethylanilin sowie Phosphorsäure-tris(dimethylamid); gegebenenfalls kann man die Reaktion auch im Gemisch dieser Lösungsmittel mit aliphatischen oder aromatischen Kohlenwasserstoffen wie n-Hexan oder Toluol durchführen. Als ausbeutesteigernde Salze, die die üblichen Nebenreaktionen unterdrücken, kommen insbesondere wasserfreie Magnesiumhalogenide wie wasserfreies Magnesiumbromid oder wasserfreie Tetraalkylammoniumhalogenide wie z.B. Tetra-n-butylammoniumchlorid in Frage. Die Reaktionstemperaturen lassen sich je nach Lösungsmittel zwischen 0° und 100 °C variieren; bevorzugt sind Temperaturen zwischen 0 °C und 60 °C. Die hierbei primär entstandenen Magnesiumalkoholate werden sodann durch Hydrolyse mit verdünnten wässrigen Säuren, wie Salzsäure, Schwefelsäure oder bevorzugt Essigsäure, oder besonders bevorzugt mit wässriger Ammoniumchloridlösung in die Alkohole übergeführt und diese nach Entfernen der wässrigen Phase, falls gewünscht, in üblicher Weise durch Extraktion, Umkristallisieren oder Chromatographie gereinigt.

Das Verfahren zur Herstellung der Ester der Formel I

$OR^2$
|
(Y= – C– in der $R^2$ Acetyl, Propionyl, n-Butyryl
|
$R^1$

oder Isobutyryl bedeutet), besteht darin, dass man die sekundären oder teriären Alkohole der Formel I

OH
|
Y= – C– mit den entsprechenden Säurechloriden
|
$R^1$

oder

Säureanhydriden in Gegenwart eines säurebindenden Mittels und gegebenenfalls in Gegenwart eines aprotischen Lösungs- oder Verdünnungsmittels sowie bevorzugt in Gegenwart eines Acylierungskatalysators bei Temperaturen zwischen 0 und 100 °C, vorzugsweise zwischen 10° und 50 °C, umsetzt. Als säurebindende Mittel können anorganische Basen wie Natriumamin oder besonders bevorzugt Pyridin in mindestens äquivalenten Mengen eingesetzt werden. Als Acylierungskatalysatoren verwendet man zweckmässigerweise Imidazol oder 4-Dimethylaminopyridin in Mengenanteilen von 0,01 bis 0,4 Äquivalenten, falls nicht bereits Pyridin anwesend ist. Als Lösungsmittel können Kohlenwasserstoffe wie Cyclohexan oder Toluol, Ether wie Diethylether, Tetrahydrofuran oder Dioxan, Ketone wie Aceton oder Diethylketon oder auch überschüssige säurebindende Amine wie Triethylamin oder Pyridin eingesetzt werden.

Das Verfahren zur Herstellung der Ether der Formel I

$OR^2$
|
(Y=–C–, in der $R^2$ $C_1$–$C_4$-Alkyl, $C_2$–$C_4$-Alkenyl
|
$R^1$

oder $C_2$–$C_4$-Alkinyl bedeutet) besteht darin, dass man die sekundären oder tertiären Alkohole der Formel I

CH
|
(Y=–C–) oder ihre Alkali- oder quartären Ammo-
|
$R^1$

niumsalze mit einem Alkylierungsmittel der Formel V

$$L{-}R^2 \qquad V,$$

gegebenenfalls in Gegenwart eines Lösungs- oder Verdünnungsmittels und gegebenenfalls in Gegenwart anorganischer oder organischer Basen und/oder eines Reaktionsbeschleunigers, zwischen 0 und 100 °C umsetzt.

Für die im obigen Verfahren erwähnten nucleophil verdrängbaren Abgangsgruppen L seien beispielsweise genannt: Halogen, vorzugsweise Chlor, Brom oder Iod; Alkylsulfat, vorzugsweise Methylsulfat; gegebenenfalls substituierte Alkylsulfonyloxyreste, vorzugsweise Methansulfonyloxy- oder Trifluormethansulfonyloxyreste; oder Arylsulfonyloxyreste, vorzugsweise der Tosylatrest.

Geeignete anorganische oder organische Basen, die gegebenenfalls auch als säurebindende Mittel in die Reaktion eingesetzt werden können, sind beispielsweise Alkali- und Erdalkalihydroxide wie Natriumhydroxid, Kaliumhydroxid, Calciumhydroxid, Alkalicarbonate wie Kalium- oder Natriumcarbonat, Alkalihydride wie Natriumhydrid, Alkali- oder Erdalkalialkoholate wie Natriummethylat, Magnesiummethylat oder Natriumisopropylat oder tertiäre Amine wie Trimethylamin, Triethylamin, N,N-Dimethylanilin, N,N-Dimethylcyclohexylamin, N-Methylpiperidin oder Pyridin. Es können aber auch andere übliche Basen verwendet werden.

Mit geeigneten Basen, z.B. Alkalihydriden wie Natriumhydrid oder Lithiumalkylen wie Butyllithium oder mit Alkali- oder Erdalkalialkoholaten wie Natriummethylat, können die Alkohole der Formel II auch in einer vorgeschalteten Umsetzung zunächst in ihre Alkoholat-Salze überführt und dann als soche zur Reaktion gebracht werden.

Zu den bevorzugten Lösungs- bzw. Verdünnungsmitteln gehören Halogenkohlenwasserstoffe wie beispielsweise Methylenchlorid, Chloroform, 1,2-Dichlorethan, Chlorbenzol, aliphatische oder aromatische Kohlenwasserstoffe wie Cyclohexan, Petrolether, Benzol, Toluol oder Xylole, Ester wie Essigsäureethylester, Amide wie Dimethylformamid, Nitrile wie Acetonitril, Sulfoxide wie Dimethylsulfoxid, Ketone wie Aceton oder Methylethylketon, Ether wie Diethylether, Tetrahydrofuran oder Dioxan oder entsprechende Gemische.

Als Reaktionsbeschleuniger kommen vorzugsweise Metallhalogenide wie Kaliumiodid, Kronenether, quartäre Ammoniumverbindungen wie Tetrabutylammoniumiodid oder Säuren oder Kombinationen dieser Reaktionsbeschleuniger in Frage.

Die erfindungsgemässen Umsetzungen werden im allgemeinen bei Temperaturen zwischen 0 und 100 °C in einem Zeitraum von 1 bis 60 Stunden durchgeführt, drucklos oder unter Druck, kontinuierlich oder diskontinuierlich.

Zur Isolierung der erfindungsgemässen Verbindungen wird nach üblichen Methoden vorgegangen. Im allgemeinen bedürfen die anfallenden Produkte keiner weiteren Reinigung, sie können aber nach bekannten Methoden wie Umkristallisation, Extraktion, Destillation oder Chromatographie weiter gereinigt werden.

Falls gewünscht, können die erfindungsgemässen Verbindungen der Formel I auch in Salze mit anorganischen oder organischen Säuren übergeführt werden, wie beispielsweise in Salze der Salzsäure, Bromwasserstoffsäure, Salpetersäure, Oxalsäure, Essigsäure, Schwefelsäure, Phosphorsäure oder Dodecylbenzolsulfonsäure. Die Wirksamkeit der Salze geht auf das Kation zurück, so dass die Wahl des Anions beliebig ist.

Ferner lassen sich die Verbindungen der Formel I nach bekannten Methoden in Metallkomplexe überführen. Das kann durch Umsetzung dieser Verbindungen mit geeigneten Metallsalzen wie beispielsweise Kupfer(II)-chlorid, Zink(II)-chlorid, Eisen(III)-chlorid, Kupfer(II)-nitrat, Mangan(II)-chlorid oder Nickel(II)-bromid erfolgen.

Die Herstellung der erfindungsgemässen Verbindungen der Formel I wird durch folgende Beispiele erläutert:

Beispiel 1

a) Herstellung des Zwischenproduktes 1-Brom-4-phenoxybutan

Eine Mischung von 329 g Phenol, 484 g trockenem Kaliumcarbonat, 756 g 1,4-Dibrombutan und 1000 ml Cyclopentanon wird unter Rühren 24 Stunden unter Rückfluss zum Sieden ehitzt. Nach Abfiltrieren der festen Bestandteile wird das Filtrat i. Vak. eingeengtt der ölige Rückstand in 1500 ml Dichlormethan aufgenommen und zehnmal mit je 200 ml 15 proz. wässriger Natriumhydroxidlösung extrahiert. Nach zweimaliger Extraktion der organischen Phase mit je 300 ml Wasser trocknet man sie über Magnesiumsulfat, filtriert und engt i. Vak. ein. Aus dem Rückstand destilliert man bei 92 bis 98 °C/0,4 mbar 395 g farbloses 1-Brom-3-phenoxybutan, das in der Vorlage erstarrt (Fp. 33 bis 36 °C).

b) Herstellung des Endproduktes

2,2,-Dimethyl-4-(1,2,4-triazol-1-yl)-8-phenoxy-octanon-(3)

Zur gerührten Suspension von 2,3 g Natriumhydrid in 20 ml Dimethylformamid (DMF) tropft man unter einer trockenen Stickstoffatmosphäre eine Lösung von 14,3 g 2,2-Dimethyl-4-(1,2,4-triazol-1-yl)-butanon-(3) (siehe DE-OS 2 638 470) in 20 ml DMF, wobei die Reaktionstemperatur durch Kühlung zwischen 20° und 30 °C gehalten wird und rührt anschliessend noch 20 Stunden bei Raumtemperatur. Dann tropft man unter weiterem Rühren und Eiskühlung eine Lösung von 19,5 g 1-Brom-4-phenoxybutan in 20 ml DMF bei 5° bis 10 °C zu. Nach Beendigung der Zugabe rührt man noch 10 Stunden bei 5° bis 10 °C weiter, fügt sodann 200 ml Wasser hinzu und extrahiert das Gemisch danach zweimal mit je 100 ml Dichlormethan. Aus den vereinigten organischen Phasen gewinnt man nach Ausschütteln mit Wasser, Trocknen über Magnesiumsulfat und Entfernen des Lösungsmittels i. Vak. ein Öl, aus dem beim Anreiben mit 20 ml Diisopropylether 17 g farbloser Kristalle ausfallen, Fp 59 bis 62 °C.

Beispiel 2

2,2-Dimethyl-4-(1,2,4-triazol-1-yl)-8-phenoxy-octanol-(3)

Zur Lösung von 600 g 2,3-Dimethyl-4-(1,2,4-triazol-1-yl)-8-phenoxy-octanol-(3) in 1000 ml Methanol gibt man unter Rühren portionsweise 72 g festes Natriumborhydrid, wobei sich die Mischung auf Rückflusstemperatur erhitzt. Nach Abklingen der exothermen Reaktion wird bis zur Trockene i. Vak. eingeengt, der Rückstand mit 500 ml Wasser versetzt und dreimal mit je 300 ml Dichlormethan extrahiert. Die vereinigten organischen Phasen werden über Magnesiumsulfat getrocknet, filtriert und das Filtrat i. Vak. eingeengt. Aus dem mit 200 ml Diisopropylether versetzten Rückstand isoliert man 360 g farblose Kristalle vom Fp. 79 bis 81 °C.

Beispiel 3

2,2-Dimethyl-3-methoxy-4-(1,2,4-triazol-1-yl)-8-phenoxy-octan

Zu einer Suspension von 1,6 g Natriumhydrid in 50 ml DMF wird eine Lösung von 15,4 g 2,2-Dimethyl-4-(1,2,4-triazol-1-yl)-8-phenoxy-octanol-(3) in 50 ml DMF getropft. Anschliessend wird das Gemisch 2 Stunden lang bei Rückflusstemperatur gerührt. Nach dem Abkühlen auf Raumtemperatur werden 3,7 ml Methyliodid zugegeben. Nach fünfstündigem Rühren bei Rückflusstemperatur werden 200 ml Wasser zugegeben. Das Reaktionsgemisch wird dreimal mit je 100 ml Diethylether extrahiert, die vereinigten Extrakte dreimal mit je

50 ml Wasser gewaschen, über Magnesiumsulfat getrocknet und i. Vak. eingeengt. Der Rückstand besteht aus 2,2 g eines gelblichen Harzes, JR (Film): 2930, 2862, 1588, 1576, 1486, 1460, 1264, 1233, 1126, 1080, 1005, 745, 682, 670 $cm^{-1}$.

Beispiel 4

2,2-Dimethyl-3-acetoxy-4-(1,2,4-triazol-1-yl)-phenoxy-octan

Eine Mischung von 10 g 2,2-Dimethyl-4-(1,2,4-triazol-1-yl)-8-phenoxy-octanol-(3), 20 ml Acetanhydrid und 1 g Imidazol wird 18 Stunden auf Rückflusstemperatur erhitzt. Nach Abkühlen auf Raumtemperatur nimmt man die Mischung in 150 ml Dichlormethan auf und schüttelt sie fünfmal mit je 50 ml Wasser, fünfmal mit je 50 ml gesättigter wässriger Natriumcarbonatlösung und dann nochmals mit Wasser aus. Nach dem Einengen der organischen Phase i. Vak. erhält man 7,9 g bräunlicher Harz, JR (Film): 2938, 2860, 1730, 1588, 1576, 1487, 1462, 1361, 1264, 1225, 1162, 1128, 1011, 745, 682, 671 $cm^{-1}$.

Beispiel 5

2,2-Dimethyl-4-(1,2,4-triazol-1-yl)-6-phenoxy-hexanon-(3)

Entsprechend Beispiel 1b werden 2,6 g Natriumhydrid, 16,7 g 2,2-Dimethyl-4-(1,2,4-triazol-1-yl)-butanon-(3) und 20,1 g 1-Brom-2-phenoxy-ethan umgesetzt.

Man erhält 11,4 g, Fp. 37 bis 40 °C.

Beispiel 6

2,2,3-Trimethyl-4-(1,2,4-triazol-1-yl)-6-phenoxy-hexanol-(3)

Zu einer Lösung von 5,4 g Methylmagnesiumbromid in 28 ml Tetrahydrofuran tropft man eine Lösung von 6,0 g 2,2-Dimethyl-4-(1,2,4-triazol-1-yl)-6-phenoxy-hexanon-() in 50 ml Tetrahydrofuran und belässt die Mischung nach Abklingen der leicht exothermen Reaktion noch 24 Stunden bei Raumtemperatur. Die Mischung wird sodann in 1000 ml 10%iger wässriger Ammoniumchloridlösung gegossen und zweimal mit je 200 ml Dichlormethan extrahiert. Nach dem Waschen der organischen Phase und Trocknen über Magnesiumsulfat wird Dichlormethan i. Vak. abgedampft. Aus dem mit 10 ml Diisopropylether versetzten Rückstand isoliert man 1,5 g weisse Kristalle, Fp. 118 bis 120 °C.

Weitere Beispiele für die erfindungsgemässen Verbindungen der Formel (I) sind in der folgenden Tabelle 1 enthalten.

Tabelle 1

| Beispiel Nr. | $X_m$ | n | Z | Y | Fp.( °C) |
|---|---|---|---|---|---|
| 7 | H | 4 | CH | CO | Harz |
| 8 | H | 4 | CH | CHOH | 87–89 |
| 9 | H | 2 | N | CHOH | 84–88 |
| 10 | H | 2 | N | $C(n-C_3H_7)OH$ | |
| 11 | 4–Cl | 2 | N | CO | 62–66 |
| 12 | 4–Cl | 2 | N | CHOH | 96–100 |
| 13 | $2,4-Cl_2$ | 2 | N | CO | 56–58 |
| 14 | $2,4-Cl_2$ | 2 | N | CHOH | 101–105 |
| 15 | H | 3 | N | CO | 55–58 |
| 16 | H | 3 | N | CHOH | 79–82 |
| 17 | 4–Cl | 3 | N | CO | 59–60 |
| 18 | 4–Cl | 3 | N | CHOH | 121–126 |
| 19 | 4–Cl | 4 | N | CO | 37–38 |
| 20 | 4–Cl | 4 | N | CHOH | 76–78 |
| 21 | $3-CF_3$ | 2 | N | CO | 36–37 |
| 22 | $3-CF_3$ | 2 | N | CHOH | 89–92 |
| 23 | 2–Cl, 4–Phenyl | 2 | N | CO | 109–113 |
| 24 | 2–Cl, 4–Phenyl | 2 | N | CHOH | 105–107 |
| 25 | $3-CH_3$ | 2 | N | CO | 46–48 |
| 26 | $3-CH_3$ | 2 | N | CHOH | 104–107 |
| 27 | H | 3 | N | $C(CH_3)OH$ | 162–163 |
| 28 | 2–F | 2 | N | CO | Harz |
| 29 | 2–F | 2 | N | CHOH | 83–87 |
| 30 | 4–F | 2 | N | CO | Harz |
| 31 | 4–F | 2 | N | CHOH | 77–82 |
| 32 | 3–Cl | 2 | N | CO | 56–60 |
| 33 | 3–Cl | 2 | N | CHOH | 85–88 |
| 34 | 3–Cl | 3 | N | CO | Harz |
| 35 | 3–Cl | 3 | N | CHOH | 74 |
| 36 | $2,4-Cl_2$ | 3 | N | CO | 57 |

Tabelle 1 (Fortsetzung)

| | | | | | |
|---|---|---|---|---|---|
| 37 | 2,4–$Cl_2$ | 3 | N | CHOH | 82–84 |
| 38 | 2,4–$Cl_2$ | 4 | N | CO | 76–77 |
| 39 | 2,4–$Cl_2$ | 4 | N | CHOH | 91–93 |
| 40 | 3,5–$Cl_2$ | 2 | N | CO | 108–113 |
| 41 | 3,5–$Cl_2$ | 2 | N | CHOH | 80–84 |
| 42 | 3–$CH_3$ | 3 | N | CO | Harz |
| 43 | 3–$CH_3$ | 3 | N | CHOH | 62 |
| 44 | 3–$CF_3$ | 3 | N | CO | Harz |
| 45 | 3–$CF_3$ | 3 | N | CHOH | 65–69 |
| 46 | 2–$OCH_3$ | 2 | N | CO | Harz |
| 47 | 3–$OCH_3$ | 2 | N | CO | 90–92 |
| 48 | 3–$OCH_3$ | 2 | N | CHOH | 101–104 |
| 49 | 4–$OCH_3$ | 2 | N | CO | 69–72 |
| 50 | 4–$OCH_3$ | 2 | N | CHOH | 92–94 |
| 51 | 4–$OCH_3$ | 3 | N | CO | 84–85 |
| 52 | 4–$OCH_3$ | 3 | N | CHOH | 104–108 |
| 53 | 3,5–$(OCH_3)_2$ | 2 | N | CO | 91–95 |
| 54 | 3,5–$(OCH_3)_2$ | 2 | N | CHOH | 104–105 |
| 55 | H | 4 | N | C(n–$C_3H_7$)OH | |
| 56 | H | 5 | N | CO | 61–63 |
| 57 | H | 5 | N | CHOH | 88–91 |
| 58 | 3–$OCH_3$ | 3 | N | CO | Harz |
| 59 | 3–$OCH_3$ | 3 | N | CHOH | 69–72 |
| 60 | H | 4 | N | CHOH 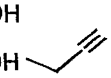 | |
| 61 | H | 4 | N | C(n–$C_4H_9$)OH | |
| 62 | H | 4 | N | C($CH_3$)OH | 114–117 |
| 63 | H | 5 | N | $CHOCH_3$ | Harz |
| 64 | H | 5 | N | CHO 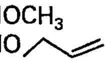 | Harz |
| 65 | 2–$OCH_3$ | 3 | N | CO | |
| 66 | 2–$OCH_3$ | 3 | N | CHOH | 53–54 |
| 67 | 2–F | 3 | N | CO | |
| 68 | 2–F | 3 | N | CHOH | 76–77 |
| 69 | 3–F | 2 | N | CO | 55–56 |
| 70 | 3–F | 2 | N | CHOH | |
| 71 | 3–F | 3 | N | CO | 49–50 |
| 72 | 3–F | 3 | N | CHOH | 89–90 |
| 73 | 4–F | 3 | N | CO | |
| 74 | 4–F | 3 | N | CHOH | 110–112 |
| 75 | 4–F | 4 | N | CHOH | 86–88 |
| 76 | 4–F | 4 | N | C($CH_3$)OH | 117–119 |
| 77 | H | 4 | CH | C($CH_3$)OH | 66–69 |
| 78 | 4–Cl | 4 | N | C($CH_3$)OH | 118–120 |
| 79 | 3–Cl | 4 | N | C($CH_3$)OH | 90 |
| 80 | 3–$CF_3$ | 4 | N | C($CH_3$)OH | 80 |

Die erfindungsgemässen Verbindungen und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zukkerrohr, Obst und Zierpflanzen im Gartenbau, sowie in Gemüsen wie Gurken, Bohnen oder Kürbisgewächsen.

Die neuen Verbindungen sind insbesondere geeignet zur Bekämpfung folgender Pflanzenkrankheiten:

Erysiphe graminis (echter Mehltau) in Getreide,
Ersiphe cichoriacearum (echter Mehltau) an Kürbisgewächsen
Podosphaera leucotricha an Äpfeln,
Uncinula necator an Reben,
Erysiphe polygony an Bohnen,
Sphaerotheca pannosa an Roden,
Puccinia-Arten an Getreide,
Phizoctonia solani an Baumwolle sowie
Helminthosporiumarten an Getreide,
Ustilago-Arten an Getreide und Zuckerrohr,
Rhynchosporium secale an Getreide und ganz besonders Venturia inaequalis (Apfelschorf).

Von den erfindungsgemässen Verbindungen der Formel I sind diejenigen als Fungizide bevorzugt, in denen $X_m$ für H–, 4–Cl–, 2,4–$Cl_2$–, und 3–$CF_3$– steht.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die erfindungsgemässen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmässige Verteilung der wirksamen Substanz gewährleisten. Die Formulierungen werden in bekannter Weise hergestellt, z.B. durch Verstrecken des Wirkstoffes mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,02 und 3 kg Wirkstoff oder mehr je ha. Die neuen Verbindungen können auch in Materialschutz u.a. zur Bekämpfung holzzerstörender Pilze wie Coniophora puteanea und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, dass man das Holz mit diesen Mitteln behandelt, beispielsweise tränkt oder anstreicht.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, vernebeln, Verstäuben, Verstreuen, Beizen oder Giessen.

Beispiele für solche Zubereitungen sind:

I. Man vermischt 90 Gewichtsteile der Verbindung des Beispiels 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in 100 000 Gewichtsteilen Wasser erhält man eine wässrige Dispersion, die 0.02 Gew.% des Wirkstoffs enthält.

III. 20 Gewichtsteile der Verbindung des Beispiels 3 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gew.% des Wirkstoffs enthält.

IV. 20 Gewichtsteile der Verbindung des Beispiels 4 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gew.% des Wirkstoffs enthält.

V. 20 Gewichtsteile der Verbindung des Beispiels 7 werden den mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffes enthält.

VI. 3 Gewichtsteile der Verbindung des Beispiels 2 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung des Beispiels 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung des Beispiels 3 werden mit 10 Teilen Natriumsalz eines Phenolsulfonsäure-harnstoff -formaldehyd -Kondensates, 2 Teilen Kieselgel und 48 Teilen Wasser innig vermischt. Man erhält eine stabile wässrige Dispersion. Durch Verdünnen mit 100 000 Gewichtsteilen Wasser erhält man eine wässrige Dispersion, die 0,04 Gew.% Wirkstoff enthält.

IX. 20 Teile der Verbindung des Beispiels 4 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die erfindungsgemässen Mittel können in diesen Anwendungsformen auch zusammen mit anderen Wirkstoffen vorliegen, wie z.B. Herbiziden, Insektiziden, Wachtumsregulatoren und Fungiziden, oder auch mit Düngemitteln vermischt und ausgebracht werden. Beim Vermischen mit Fungiziden erhält man dabei in vielen Fällen eine Vergrösserung des fungiziden Wirkungsspektrums.

Die folgenden Beispiele A und B zeigen die fungizide Wirkung der neuen Substanzen. Als Vergleichssubstanzen dienten Imazalil (1-(2'-(2'',4''-Dichlorphenyl)-2' -(2''-propenyloxy)-ethyl)-1H-imidazol) sowie das 2,2-Dimethyl-4-(1,2,4-triazol-1-yl)-5-phenyl-pentanon-(3) (DE-OS 2 638 470).

Beispiel A

Wirksamkeit geben Weizenmehltau

Blätter von in Töpfen gewachsenen Weizenkeimlingen der Sorte «Jubilar» werden mit wässrigen Emulsionen aus 80% (Gew.%) Wirkstoff und 20% Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschliessend im Gewächshaus bei Temperaturen zwischen 20 und 22 °C und 75 bis 80% relativer Luftfeuchtigkeit aufgestellt. Nach 10 Tagen wird das Ausmass der Mehltauentwicklung ermittelt.

In diesem Versuch zeigten beispielsweise besonders die Substanzen der Beispiele 1, 2, 9, 11–13, 15–22, 24, 26, 29–34, 39, 41, 45, 47, 49 und 50 eine bessere Wirkung als Imazalil.

Beispiel B

Wirksamkeit gegen Weizenbraunrost

Blätter von in Töpfen gewachsenen Weizensämlingen der Sorte «Caribo» werden mit Sporen des Braunrostes (Puccinia recondita) bestäubt. Danach werden die Töpfe für 24 Stunden bei 20 bis 22 °C in eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschliessend mit 0,025–0,006- und 0,0015-%igen (Gew.%) wässrigen Spritzbrühen, die 80% Wirkstoff und 20% Ligninsulfonat in der Trockensubstanz enthalten, tropfnass gespritzt. Nach dem Antrocknen des Spritzbelages werden die Versuchspflanzen im Gewächshaus bei Temperaturen zwischen 20 und 22 °C und 65 bis 70% relativer Luftfeuchte aufgestellt. Nach 8 Tagen wird das Ausmass der Rostpilzentwicklung auf den Blättern ermittelt.

In diesem Versuch zeigten beispielsweise besonders die Substanzen der Beispiele 2, 16–20, 22, 35, 38, 43, 44 und 45 eine bessere Wirkung als 2,2-Dimethyl-4-(1,2,4-triazol-1-yl)-5-phenyl-pentanon-(3).

Die neuen erfindungsgemässen Wirkstoffe greifen auch in den Metabolismus der Pflanzen ein und können deshalb als Wachstumsregulatoren eingesetzt werden.

Für die Wirkungsweise von Pflanzenwachstumsregulatoren gilt nach den bisherigen Erfahrungen, dass ein Wirkstoff eine oder auch mehrere verschiedenartige Wirkungen auf Pflanzen ausüben kann.

Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren (Samenbeize, Bodenbehandlung oder Blattapplikation),

d) von den geoklimatischen Faktoren, z.B. Sonnenscheindauer, Durchschnittstemperatur, Niederschlagsmenge,

e) von der Bodenbeschaffenheit (einschliesslich Düngung),

f) von der Formulierung bzw. Anwendungsform des Wirkstoffs und schliesslich

g) von den angewendeten Konzentrationen bzw. Aufwandmengen der aktiven Substanz.

In jedem Falle sollen Wachstumsregulatoren die Kulturpflanzen in gewünschter Weise positiv beeinflussen.

Aus der Reihe der verschiedenartigen Anwendungsmöglichkeiten der erfindungsgemässen Pflanzenwachtumsregulatoren im Pflanzenanbau, in der Landwirtschaft und im Gartenbau, werden einige nachstehend erwähnt.

A. Mit den erfindungsgemäss verwendbaren Verbindungen lässt sich das vegetative Wachstum der Pflanzen stark hemmen, was sich insbesondere in einer Reduzierung des Längenwachstums äussert. Die behandelten Pflanzen weisen demgemäss einen gedrungenen Wuchs auf; ausserdem ist eine dunklere Blattfärbung zu beobachten.

Als vorteilhaft für die Praxis erweist sich z.B. die Verringerung des Grasbewuchses an Strassenrändern, Kanalböschungen und auf Rasenflächen wie Park-, Sport- und Obstanlagen, Zierrasen und Flugplätzen, so dass der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des «Lagerns» (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben zugunsten des Blattwachstums gehemmt werden soll.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird. Ferner kann so wegen der relativ geringen Blatt- bzw. Pflanzenmasse dem Befall mit verschiedenen, insbesondere pilzlichen Krankheiten vorgebeugt werden.

Mit Wachstumshemmern lässt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide ist es vorteilhaft, wenn die Bestände durch Behandlung mit erfindungsgemässen Verbindungen im Herbst zwar gut bestockt werden, aber nicht zu üppig in den Winter hineingehen. Dadurch kann der erhöhten Frostempfindlichkeit und – wegen der relativ geringen Blatt- bzw. Pflanzenmasse – dem Befall mit verschiedenen Krankheiten (z.B. Pilzkrankheit) vorgebeugt werden.

Die Hemmung des vegetativen Wachstums ermöglicht ausserdem bei vielen Kulturpflanzen eine dichtere Bepflanzung, so dass ein Mehrertrag bezogen auf die Bodenfläche erzielt werden kann. Die erfindungsgemässen Verbindungen eignen sich besonders zur Hemmung des vegetativen Wachstums bei einem breiten Spektrum von Kulturpflanzen wie Weizen, Roggen, Hafer, Mais, Sonnenblumen, Erdnüssen, Tomaten, verschiedenen Zierpflanzen, wie Chrysanthemen, Poinsettien und Hibiskus, Baumwolle und insbesondere bei Soja, Raps, Gerste, Reis und Gräsern.

B. Mit den neuen Wirkstoffen lassen sich Mehrerträge sowohl an Pflanzenteilen als auch an Pflanzeninhaltsstoffen erzielen. So ist es beispielsweise möglich, das Wachstum grösserer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluss zu stimulieren.

Dabei können die neuen Stoffe Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schliesslich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heisst die Förderung der Ausbildung von Trenngewebe zwischen Frucht- bzw. Blatt und Sprossteil der Pflanze ist auch für ein gut kontrollierbares Entblättern der Bäume wesentlich.

Die erfindungsgemässen Wirkstoffe können den Kulturpflanzen sowohl vom Samen her (als Saatgutbeizmittel) als auch über den Boden, d.h. durch die Wurzel sowie – besonders bevorzugt – durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,001 bis 12 kg/ha bevorzugt 0,01 bis 3 kg/ha als ausreichend zu betrachten.

Die erfindungsgemässen Verbindungen können für ihre Anwendung als Pflanzenwachstumsregulatoren in die üblichen Formulierungen überführt werden, entsprechend dem oben beschriebenen Anwendungsformen als Fungizide. Die so erhaltenen Formulierungen bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Die erfindungsgemässen wachstumsregulierenden Mittel können in diesen Anwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Wachstumsregulatoren erhält man dabei in vielen Fällen eine Vergrösserung des Wirkungsspektrums. Bei einer Anzahl solcher Wachstumsregulatormischungen treten auch synergistische Effekte auf, d.h. die Wirksamkeit des Kombinationsproduktes ist grösser als die addierten Wirksamkeiten der Einzelkomponenten.

Von den erfindungsgemässen Verbindungen der Formel I sind diejenigen als Pflanzenwachstumsregulatoren bevorzugt, bei denen n für die Zahlen 2 oder 3, besonders bevorzugt für 2, Z für N und Y für die Gruppen CO oder CHOH steht.

Zur Bestimmung der wachstumsregulierenden Eigenschaften der Prüfsubstanzen wurden Testpflanzen auf ausreichend mit Nährstoffen versorgtem Kultursubstrat in Kunststoffgefässen von ca. 12,5 cm Durchmesser angezogen.

Im Vorauflaufverfahren wurden die Testsubstanzen in wässriger Aufbereitung am Tage der Einsaat auf das Saatbett gegossen.

Im Nachauflaufverfahren wurden die zu prüfenden Substanzen in wässriger Aufbereitung auf die Pflanzen gesprüht. Die beobachtete wachstumsregulierende Wirkung wurde bei Versuchsende durch Wuchshöhenmessung belegt. Die so gewonnenen Messwerte wurden zur Wuchshöhe der unbehandelten Pflanzen in Relation gesetzt. Als Vergleichssubstanzen dienten 3-(1,2,4-Triazol-1-yl)-1-(4- chlorphenyl)-4,4- dimethylpentanon- (1) (DE-OS 2 739 352) bzw. (2-Chlorethyl)-trimethylammoniumchlorid (CCC).

In diesen Versuchen, die an Sommergerste, Reis, Rasen, Sommerraps und Soja durchgeführt wurden, zeigten beispielsweise insbesondere die Substanzen der Beispiele 1, 9, 12, 13, 14, 16, 17, 19, 22, 26, 27 und 29 bis 35 eine bessere Wirkung als die Vergleichssubstanzen.

**Patentansprüche für die Vertragsstaaten: BE, CH, DE, FR, GB, IT, LI, NL, SE**

1. Azolverbindungen der Formel I

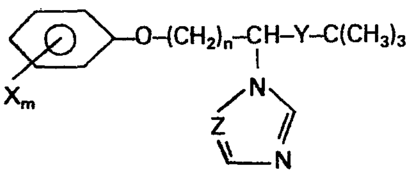

(I),

in welcher

X für Wasserstoff, Halogen, $C_1$–$C_4$–Alkyl, $C_1$–$C_4$–Alkyloxy, Trifluormethyl oder Phenyl steht und m eine ganze Zahl von 1 bis 5 ist, wobei die einzelnen Gruppen X gleich oder verschieden sind, wenn m grösser als 1 ist,

n eine ganze Zahl von 2 bis 5 ist,

Z für N oder Ch steht und

Y für CO oder die Gruppe $CR^1OR^2$ steht, in welcher $R^1$ Wasserstoff oder $C_1$–$C_4$–Alkyl bedeutet und $R^2$ Wasserstoff oder $C_1$–$C_4$–Alkyl, $C_2$–$C_4$–Alkenyl, $C_2$–$C_4$–Alkinyl oder $C_1$–$C_4$–Alkanoyl bedeutet, sowie deren für Pflanzen verträgliche Säureadditionssalze und Metall-Komplexe.

2. Verfahren zur Herstellung der Azolverbindungen der Formel I gemäss Anspruch 1, dadurch gekennzeichnet, dass man Ketone der Formel II

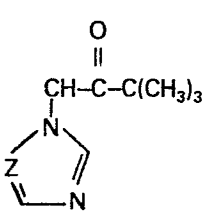

(II),

in der Z die angegebenen Bedeutungen hat, oder deren Alkalienolate mit einem ω-Aryloxyalkylhalogenid der Formel III

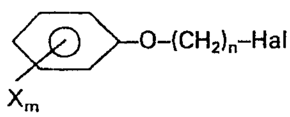

(III),

in der X, m und n die unter Anspruch 1 angegebenen Bedeutungen haben und Hal für Chlor, Brom oder Iod steht, gegebenenfalls in Gegenwart eines Lösungs- oder Verdünnungsmittels und/oder anorganischer oder organischer Basen bei Temperaturen zwischen 0 und 100 °C, umsetzt und die so erhaltenen Verbindungen der Formel I, in denen Y eine CO-Gruppe darstellt, gegebenenfalls anschliessend durch Einwirkung eines komplexen Hydrids oder durch Einwirkung von Wasserstoff in Gegenwart eines Hydrierungskatalysators und gegebenenfalls in Gegenwart eines Lösungs- oder Verdünnungsmittels bei Temperaturen zwischen 0 und 100 °C zu sekundären Alkoholen der Formel I, in denen Y eine CHOH-Gruppe darstellt, reduziert oder mit einer Grignardverbindung der Formel IV

$R^1$–MgHal IV,

in der $R^1$ für $C_1$–$C_4$–Alkyl steht und in der Hal Chlor, Brom oder Iod bedeutet, gegebenenfalls in Gegenwart eines Lösungs- oder Verdünnungsmittels und gegebenenfalls in Gegenwart eines Magnesium- oder Tetraalkylammoniumhalogenids bei Temperaturen zwischen 0 und 100 ° C umsetzt und die so entstandenen Alkoholate anschliessend zu den tertiären Alkoholen hydrolysiert und die so erhaltenen sekundären oder tertiären Alkohole – falls gewünscht – mit einem $C_1$–$C_4$–Alkanoylchlorid oder einem $C_1$–$C_4$–Alkanoylanhydrid gegebenenfalls in Gegenwart eines Lösungs- oder Verdünnungsmittels und/oder einer anorganischen oder organischen Base und/oder eines Acylierungskatalysators bei Temperaturen zwischen 0 und 100 °C umsetzt oder diese oder deren Alkali- oder quartären Ammoniumsalze mit einem Alkylierungsmittel der Formel V

L–$R^2$ V,

in der $R^2$ $C_1$–$C_4$–Alkyl, $C_2$–$C_4$–Alkenyl oder $C_2$–$C_4$–Alkinyl bedeutet und L für eine nucleophil verdrängbare Abgangsgruppe steht, gegebenenfalls in Gegenwart eines Lösungs- oder Verdünnungsmittels und/oder anorganischer oder organischer Basen und/oder eines Reaktionsbeschleunigers bei Temperaturen zwischen 0 und 100 °C umsetzt und die so erhaltenen Verbindungen der Formel I gegebenenfalls anschliessend in ihre für Pflanzen verträglichen Säureadditionssalze oder Metallkomplexe überführt.

3. Pflanzenbehandlungsmittel, enthaltend eine Verbindung gemäss Anspruch 1.

4. Verfahren zur Behandlung von Pflanzen, dadurch gekennzeichnet, dass man mindestens eine Verbindung der Formel I gemäss Anspruch 1 auf diese einwirken lässt.

5. Verfahren zur vorbeugenden Behandlung von Pflanzen, dadurch gekennzeichnet, dass man mindestens eine Verbindung der Formel I gemäss Anspruch 1 auf Pflanzen, deren Saatgüter oder deren Anbauflächen einwirken lässt.

6. Verfahren zur Herstellung von Pflanzenbehandlungsmitteln, dadurch gekennzeichnet, dass man eine oder mehrere Verbindungen gemäss Anspruch 1 mit festen oder flüssigen Trägerstoffen sowie gegebenenfalls mit einem oder mehreren oberflächenaktiven Mitteln mischt.

**Patentansprüche für den Vertragsstaat: AT**

1. Verfahren zur Herstellung von Azolverbindungen der Formel I

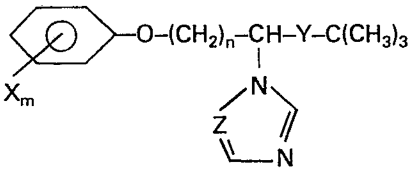

(I),

in welcher

X für Wasserstoff, Halogen, $C_1$–$C_4$–Alkyl, $C_1$–$C_4$–Alkyloxy, Trifluormethyl oder Phenyl steht und m eine ganze Zahl von 1 bis 5 ist, wobei die einzelnen Gruppen X gleich oder verschieden sind, wenn m grösser als 1 ist,

n eine ganze Zahl von 2 bis 5 ist,

Z für N oder CH steht und

Y für CO oder die Gruppe $CR^1OR^2$ steht, in welcher $R^1$ Wasserstoff oder $C_1$–$C_4$–Alkyl bedeutet und $R^2$ Wasserstoff, $C_1$–$C_4$–Alkyl, $C_2$–$C_4$–Alkenyl, $C_2$–$C_4$–Alkinyl oder $C_1$–$C_4$–Alkanoyl bedeutet, sowie von deren für Pflanzen verträglichen Säureadditionssalzen und Metall-Komplexen, dadurch gekennzeichnet, dass man Ketone der Formel II

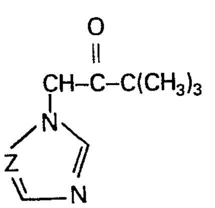

(II),

in der Z die angegebenen Bedeutungen hat, oder deren Alkalienolate mit einem ω-Aryloxyalkylhalogenid der Formel III

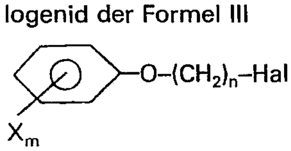

(III),

in der X, m und n die unter Anspruch 1 angegebenen Bedeutungen haben und Hal für Chlor, Brom oder Iod steht, gegebenenfalls in Gegenwart eines Lösungs- oder Verdünnungsmittels und/oder anorganischer oder organischer Basen bei Temperaturen zwischen 0 und 100 °C, umsetzt und die so erhaltenen Verbindungen der Formel I, in denen Y eine CO-Gruppe darstellt, gegegenenfalls anschliessend durch Einwirkung eines komplexen Hydrids oder durch Einwirkung von Wasserstoff in Gegenwart eines Hydrierungskatalysators und gegebenenfalls in Gegenwart eines Lösungs- oder Verdünnungsmittels bei Temperaturen zwischen 0 und 100 °C zu sekundären Alkoholen der Formel I, in denen Y eine CHOH-Gruppe darstellt, reduziert oder mit einer Grignardverbindung der Formel IV

$R^1$–MgHal IV,

in der $R^1$ für $C_1$–$C_4$–Alkyl steht und in der Hal Chlor, Brom oder Iod bedeutet, gegebenenfalls in Gegenwart eines Lösungs- oder Verdünnungsmittels und gegebenenfalls in Gegenwart eines Magnesium- oder Tetraalkylammoniumhalogenids bei Temperaturen zwischen 0 und 100 °C umsetzt und die so entstandenen Alkoholate anschliessend zu den tertiären Alkoholen hydrolysiert und die so erhaltenen sekundären oder tertiären Alkohole – falls gewünscht – mit einem $C_1$–$C_4$–Alkanoylchlorid oder einem $C_1$–$C_4$–Alkanoylanhydrid gegebenenfalls in Gegenwart eines Lösungs- oder Verdünnungsmittels und/oder einer anorganischen oder organischen Base und/oder eines Acylierungskatalysators bei Temperaturen zwischen 0 und 100 °C umsetzt oder diese oder deren Alkali- oder quartären Ammoniumsalze mit einem Alkylierungsmittel der Formel V

L–$R^2$ V,

in der $R^2$ $C_1$–$C_4$–Alkyl, $C_2$–$C_4$–Alkenyl oder $C_2$–$C_4$–Alkinyl bedeutet und L für eine nucleophil verdrängbare Abgangsgruppe steht, gegebenenfalls in Gegenwart eines Lösungs- oder Verdünnungsmittels und/oder anorganischer oder organischer Basen und/oder eines Reaktionsbeschleunigers bei Temperaturen zwischen 0 und 100 °C umsetzt und die so erhaltenen Verbindungen der Formel I gegebenenfalls anschliessend in ihre für Pflanzen verträglichen Säureadditionssalze oder Metallkomplexe überführt.

2. Pflanzenbehandlungsmittel, enthaltend eine Verbindung gemäss Anspruch 1.

3. Verfahren zur Behandlung von Pflanzen, dadurch gekennzeichnet, dass man mindestens eine Verbindung der Formel I gemäss Anspruch 1 auf diese einwirken lässt.

4. Verfahren zur vorbeugenden Behandlung von Pflanzen, dadurch gekennzeichnet, dass man mindestens eine Verbindung der Formel I gemäss Anspruch 1 auf Pflanzen, deren Saatgüter oder deren Anbauflächen einwirken lässt.

5. Verfahren zur Herstellung von Pflanzenbehandlungsmitteln, dadurch gekennzeichnet, dass man eine oder mehrere Verbindungen gemäss Anspruch 1 mit festen oder flüssigen Trägerstoffen sowie gegebenenfalls mit einem oder mehreren oberflächenaktiven Mitteln mischt.

**Revendication pour les Etats contractants: BE, CH, DE, FR, GB, IT, LI, NL, SE**

1. Dérivés d'azoles de la formule I

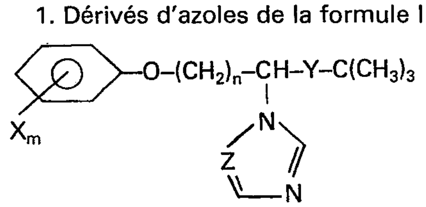

(I),

dans laquelle

X désigne un atome d'hydrogène ou d'halogène, un radical alcoyle en $C_1$ à $C_4$ ou un groupe alcoyl (en $C_1$ à $C_4$)-oxy, trifluorométhyle ou phényle et m est un nombre entier de 1 à 5, les divers groupes X pouvant être identiques ou différents, lorsque m est supérieur à 1;

n est un nombre entier valant de 2 à 5;

Z représente N ou CH et

Y représente CO ou un groupe $CR^1OR^2$, où $R^1$ désigne un atome d'hydrogène ou un radical alcoyle en $C_1$ à $C_4$ et $R^2$ un atome d'hydrogène ou un radical alcoyle en $C_1$ à $C_4$, alcényle en $C_2$ à $C_4$, alcynyle en $C_2$ à $C_4$ ou alcanoyle en $C_1$ à $C_4$, ainsi que les sels d'addition d'acides et complexes métalliques non phytotoxiques de ces dérivés.

2. Procédé de préparation des dérivés d'azoles de la formule I selon la revendication 1, caractérisé en ce que l'on fait réagir des cétones de la formule II

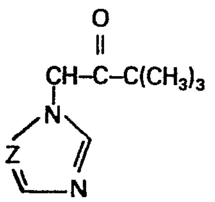

(II),

dans laquelle Z possède les significations définies, ou leurs énolates d'alcali, avec un halogénure d'ω-aryloxyalcoyle de la formule III

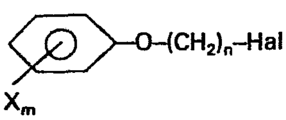

(III),

dans laquelle X, m et n possèdent les significations définies dans la revendication 1 et Hal désigne du chlore, du brome ou de l'iode, à des températures comprises entre 0 et 100 °C, éventuellement en présence d'un solvant ou d'un diluant et (ou) de bases minérales ou organiques, puis on réduit le cas échéant les composés obtenus de la formule I, dans lesquels Y est un groupe –CO, par un hydrure complexe ou par l'hydrogène en présence d'un catalyseur d'hydrogénation, à des températures comprises entre 0 et 100 °C et éventuellement en présence d'un solvant ou d'un diluant, en alcools secondaires de la formule I, dans lesquels Y est un groupe –CHOH, ou on les fait réagir à des températures comprises entre 0 et 100 °C, éventuellement en présence d'un solvant ou d'un diluant et éventuellement en présence d'un halogénure de magnésium ou de tétraalcoylammonium, avec un réactif de Grignard de la formule IV

$R^1$–MgHal (IV),

dans laquelle $R^1$ désigne un radical alcoyle en $C_1$ à $C_4$ et Hal du chlore, du brome ou de l'iode, puis on hydrolyse les alcoolates formés en alcools tertiaires et, le cas échéant, on fait réagir les alcools secondaires ou tertiaires ainsi obtenus à des températures comprises entre 0 et 100 °C avec un chlorure d'alcanoyle en $C_1$ à $C_4$ ou avec un anhydride d'alcanoyle en $C_1$ à $C_4$, éventuellement en présence d'un solvant ou d'un diluant et (ou) d'une base minérale ou organique et (ou) d'un catalyseur d'acylation, ou on fait réagir ces alcools ou leurs sels de métal alcalin ou d'ammonium quaternaire, à des températures comprises entre 0 et 100 °C, avec un agent d'alcoylation de la formule V

L–$R^2$ (V),

dans laquelle $R^2$ désigne un radical alcoyle en $C_1$ à $C_4$, alcényle en $C_2$ à $C_4$ ou alcynyle en $C_2$ à $C_4$ et L un groupe pouvant être éliminé par un clivage nucléophile, éventuellement en présence d'un solvant ou d'un diluant et (ou) de bases inorganiques ou organiques et (ou) d'un accélérateur de la réaction, les composés de la formule I obtenus pouvant ensuite être transformés, si on le désire, en leurs sels d'addition d'acides ou complexes métalliques non phytotoxiques.

3. Composition pour le traitement de plantes, contenant un dérivé selon la revendication 1.

4. Procédé de traitement de plantes, caractérisé en ce que l'on fait agir sur celles-ci au moins un dérivé de la formule I selon la revendication 1.

5. Procédé de traitement préventif de plantes, caractérisé en ce que l'on fait agir au moins un dérivé de la formule I selon la revendication 1 sur les plantes, les semences ou les surfaces de culture.

6. Procédé de préparation de compositions pour le traitement de plantes, caractérisé en ce que l'on mélange un ou plusieurs dérivés selon la revendication 1 avec des véhicules ou supports liquides ou solides et éventuellement avec un ou plusieurs agents tensio-actifs.

**Revendications pour l'Etat contractant: AT**

1. Procédé de préparation de dérivés d'azoles de la formule I

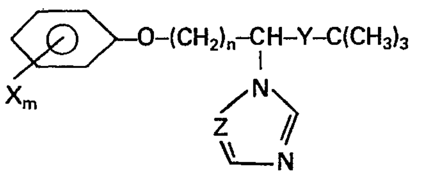

(I),

dans laquelle

X désigne un atome d'hydrogène ou d'halogène, un radical alcoyle en $C_1$ à $C_4$ ou un groupe alcoyl (en $C_1$ à $C_4$)-oxy, trifluorométhyle ou phényle et m est un nombre entier de 1 à 5, les divers groupes X pouvant être identiques ou différents, lorsque m est supérieur à 1;

n est un nombre entier valant de 2 à 5;

Z représente N ou CH et

Y représente CO ou un groupe $CR^1OR^2$, où $R^1$ désigne un atome d'hydrogène ou un radical alcoyle en $C_1$ à $C_4$ et $R^2$ un atome d'hydrogène ou un radical alcoyle en $C_1$ à $C_4$, alcényle en $C_2$ à $C_4$, alcynyle en $C_2$ à $C_4$ ou alcanoyle en $C_1$ à $C_4$, ainsi que les sels d'addition d'acides et complexes métalliques non phytotoxiques de ces dérivés, caractérisé en ce que l'on fait réagir des cétones de la formule II

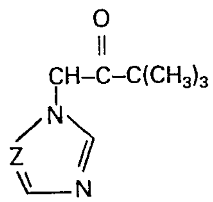 (II),

dans laquelle Z possède les significations définies, ou leurs énolates d'alcali, avec un halogénure d'ω-aryloxyalcoyle de la formule III

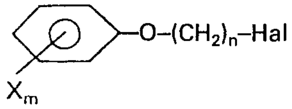 (III),

dans laquelle X, m et n possèdent les significations définies ci-dessus et Hal représente un atome de chlore, du brome ou de l'iode, à des températures comprises entre 0 et 100 °C, éventuellement en présence d'un solvant ou d'un diluant et(ou) de bases minérales ou organiques, puis on réduit le cas échéant les composés obtenus de la formule I, dans lesquels Y est un groupe –CO, par un hydrure complexe ou par l'hydrogène en présence d'un catalyseur d'hydrogénation, à des températures comprises entre 0 et 100 °C et éventuellement en présence d'un solvant ou d'un diluant, en alcools secondaires de la formule I, dans lesquels Y est un groupe –CHOH, ou on les fait réagir à des températures comprises entre 0 et 100 °C, éventuellement en présence d'un solvant ou d'un diluant et éventuellement en présence d'un halogénure de magnésium ou de tétraalcoylammonium, avec un réactif de Grignard de la formule IV

$R^1$–MgHal (IV),

dans laquelle $R^1$ désigne un radical alcoyle en $C_1$ à $C_4$ et Hal du chlore, du brome ou de l'iode, puis on hydrolyse les alcoolates formés en alcools tertiaires et, le cas échéant, on fait réagir les alcools secondaires ou tertiaires ainsi obtenus à des températures comprises entre 0 et 100 °C avec un chlorure d'alcanoyle en $C_1$ à $C_4$ ou avec un anhydride d'alcanoyle en $C_1$ à $C_4$, éventuellement en présence d'un solvant ou d'un diluant et (ou) d'une base minérale ou organique et (ou) d'un catalyseur d'acylation, ou on fait réagir ces alcools ou leurs sels de métal alcalin ou d'ammonium quaternaire, à des températures comprises entre 0 et 100 °C, avec un agent d'alcoylation de la formule V

L–$R^2$ (V),

dans laquelle $R^2$ désigne un radical alcoyle en $C_1$ à $C_4$, alcényle en $C_2$ à $C_4$ ou alcynyle en $C_2$ à $C_4$ et L un groupe pouvant être éliminé par un clivage nucléophile, éventuellement en présence d'un solvant ou d'un diluant et(ou) de bases inorganiques ou organiques et(ou) d'un accélérateur de la réaction, les composés de la formule I obtenus pouvant ensuite être transformés, si on le désire, en leurs sels d'addition d'acides ou complexes métalliques non phytotoxiques.

2. Composition pour le traitement de plantes, contenant un dérivé selon la revendication 1.

3. Procédé de traitement de plantes, caractérisé en ce que l'on fait agir sur celles-ci au moins un dérivé de la formule I selon la revendication 1.

4. Procédé de traitement préventif de plantes, caractérisé en ce que l'on fait agir au moins un dérivé de la formule I selon la revendication 1 sur les plantes, les semences ou les surfaces de culture.

5. Procédé de préparation de compositions pour le traitement de plantes, caractérisé en ce que l'on mélange un ou plusieurs dérivés selon la revendication 1 avec des véhicules ou supports liquides ou solides et éventuellement avec un ou plusieurs agents tensio-actifs.

**Claims for the Contracting states: BE, CH, DE, FR, GB, IT, LI, NL, SE**

1. An azole compound of the formula I

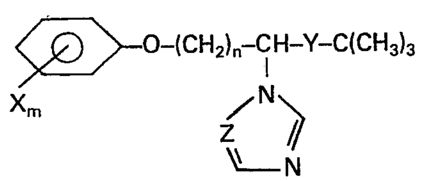 (I),

where X is hydrogen, halogen, $C_1$–$C_4$–alkyl, $C_1$–$C_4$–alkoxy, trifluoromethyl or phenyl and m is an integer from 1 to 5, and, if m is greater than 1, the X's can be identical or different, n is an integer from 2 to 5, Z is N or CH and Y is CO or $CR^1OR^2$, where $R^1$ is hydrogen or $C_1$–$C_4$–alkyl and $R^2$ is hydrogen, $C_1$–$C_4$–alkyl, $C_2$–$C_4$–alkenyl, $C_2$–$C_4$–alkynyl or $C_1$–$C_4$–alkanoyl, as well as its crop-tolerated addition salts with acids and its metal complex salts.

2. A process for manufacturing an azole compound of the formula I as claimed in claim 1, wherein a ketone of the formula II

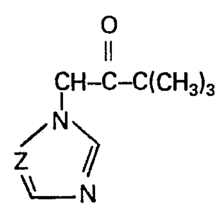 (II),

where Z has the above meanings, or an alkali metal enolate thereof, is reacted with a ω-aryloxyalkyl halide of the formula III

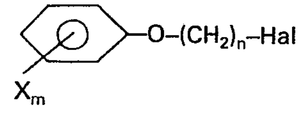 (III),

where X, m and n have the meanings given in claim 1 and Hal is chlorine, bromine or iodine, in the presence or absence of a solvent or diluent and/or of an inorganic or organic base, at from 0 to 100 °C, after which the resulting compound of the formula I, where Y is a CO group, can, if desired, be reduced, by treatment with a complex hydride or by treatment with hydrogen in the presence of a hydrogenation catalyst, in the presence or absence of a solvent or diluent, at from 0 to 100 °C, to a secondary alcohol of the formula I, where Y is a CHOH group, or be reacted with a Grignard compound of the formula IV

$R^1$–MgHal IV,

where $R^1$ is $C_1$–$C_4$-alkyl and Hal is chlorine, bromine or iodine, in the presence or absence of a solvent or diluent and in the presence or absence of a magnesium halide or tetraalkylammonium halide, at from 0 to 100 °C, the resulting alcoholate then being hydrolyzed to the tertiary alcohol and the secondary or tertiary alcohol thus obtained can, if desired, be reacted with a $C_1$–$C_4$-alkanoyl chloride or a $C_1$–$C_4$-alkanoyl anhydride to form the corresponding ester, in the presence or absence of a solvent or diluent and/or of an inorganic or organic base and/or of an acylation catalyst, at from 0 to 100 °C, or it or its alkali metal salts or quaternary ammonium salts can, for the preparation of the corresponding ethers, be reacted with an alkylating agent of the formula V

L–$R^2$ V,

where $R^2$ is $C_1$–$C_4$-alkyl, $C_2$–$C_4$-alkenyl or $C_2$–$C_4$-alkynyl and L is a nucleophilically displaceable leaving group, in the presence or absence of a solvent or diluent and/or of an inorganic or organic base and/or of a reaction accelerator, at from 0 to 100 °C, and the compounds of the formula I thus obtained are, if desired, subsequently converted to their crop-tolerated acid addition salts or metal complexes.

3. A plant treatment agent containing a compound as claimed in claim 1.

4. A process for treating plants, wherein at least one compound of the formula I as claimed in claim 1 is allowed to act thereon.

5. A process for the prophylactic treatment of plants, wherein at least one compound of the formula I as claimed in claim 1 is allowed to act on plants, their seed, or the land on which they are grown.

6. A process for manufacturing plant treatment agents, wherein one or more compounds as claimed in claim 1 are mixed with solid or liquid carriers and, if desired, with one or more surfactants.

**Claims for the Contracting state: AT**

1. A process for manufacturing an azole compound of the formula I

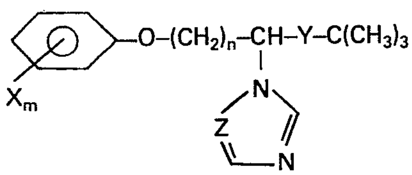

(I),

where X is hydrogen, halogen, $C_1$–$C_4$-alkyl, $C_1$–$C_4$-alkoxy, trifluoromethyl or phenyl and m is an integer from 1 to 5, and, if m is greater than 1, the X's can be identical or different, n is an integer from 2 to 5, Z is N or CH and Y is CO or $CR^1OR^2$, where $R^1$ is hydrogen or $C_1$–$C_4$-alkyl and $R^2$ is hydrogen, $C_1$–$C_4$-alkyl, $C_2$–$C_4$-alkenyl, $C_2$–$C_4$-alkynyl or $C_1$–$C_4$-alkanoyl, as well as its crop-tolerated addition salts with acids and its metal complex salts, wherein a ketone of the formula II

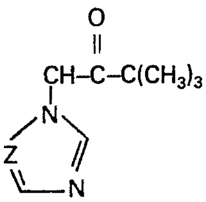

(II),

where Z has the above meanings, or an alkali metal enolate thereof, is reacted with a ω-aryloxyalkyl halide of the formula III

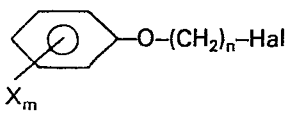

(III),

where X, m and n have the meanings given in claim 1 and Hal is chlorine, bromine or iodine, in the presence or absence of a solvent or diluent and/or of an inorganic or organic base, at from 0 to 100 °C, after which the resulting compound of the formula I, where Y is a CO group, can, if desired, be reduced, by treatment with a complex hydride or by treatment with hydrogen in the presence of a hydrogenation catalyst, in the presence or absence of a solvent or diluent, at from 0 to 100 °C, to a secondary alcohol of the formula I, where Y is a CHOH group, or be reacted with a Grignard compound of the formula IV

$R^1$–MgHal IV,

where $R^1$ is $C_1$–$C_4$-alkyl and Hal is chlorine, bromine or iodine, in the presence or absence of a solvent or diluent and in the presence or absence of a magnesium halide or tetraalkylammonium halide, at from 0 to 100 °C, the resulting alcoholate then bein hydrolyzed to the tertiary alcohol and the secondary or tertiary alcohol thus obtained can, if desired, be reacted with a $C_1$–$C_4$-alkanoyl chloride or a $C_1$–$C_4$-alkanoyl anhydride to form the corresponding ester, in the presence or absence of a solvent or diluent and/or of an inorganic or organic base and/or of an acylation catalyst, at from 0 to 100 °C or it or its alkali metal salts or quaternary ammonium salts can, for the preparation of the corresponding ethers, be reacted with an alkylating agent of the formula V

$$L-R^2 \qquad V,$$

where $R^2$ is $C_1$–$C_4$–alkyl, $C_2$–$C_4$–alkenyl or $C_2$–$C_4$–alkynyl and L is a nucleophilically displaceable leaving group, in the presence or absence of a solvent or diluent and/or of an inorganic or organic base and/or of a reaction accelerator, at from 0 to 100 °C, and the compounds of the formula I thus obtained are, if desired, subsequently converted to their crop-tolerated acid addition salts or metal complexes.

2. A plant treatment agent containing a compound according to claim 1.

3. A process for treating plants, wherein at least one compound of the formula I according to claim 1 is allowed to act thereon.

4. A process for the prophylactic treatment of plants, wherein at least one compound of the formula I according to claim 1 is allowed to act on plants, their seed, of the land on which they are grown.

5. A process for manufacturing plant treatment agents, wherein one or more compounds according to claim 1 are mixed with solid or liquid carriers and, if desired, with one or more surfactants.